Europäisches Patentamt

European Patent Office

Office européen des brevets

(11) Publication number: **0 241 201**
**A2**

## EUROPEAN PATENT APPLICATION

(21) Application number: **87302769.2**

(51) Int. Cl.⁴: **A61K 37/64**

(22) Date of filing: **31.03.87**

(30) Priority: **07.04.86 US 848528**

(43) Date of publication of application:
**14.10.87 Bulletin 87/42**

(84) Designated Contracting States:
**AT BE CH DE ES FR GB IT LI LU NL SE**

(71) Applicant: **MERCK & CO. INC.**
**126, East Lincoln Avenue P.O. Box 2000**
**Rahway New Jersey 07065-0900(US)**

(72) Inventor: **Sweet, Charles S.**
**741 Cowpath Road**
**Telford Pennsylvania 18969(US)**

(74) Representative: **Hesketh, Alan, Dr.**
**European Patent Department Merck & Co.,**
**Inc. Terlings Park Eastwick Road**
**Harlow Essex, CM20 2QR(GB)**

(54) Angiotensin converting enzyme inhibitors useful in prolonging survival of mammalians with congestive heart failure.

(57) Angiotensin converting enzyme (ACE) inhibitors have been found useful in prolonging the survival rate of mammalians with congestive heart failure (CHF), particularly those who had a large infarct.

EP 0 241 201 A2

# ANGIOTENSIN CONVERTING ENZYME INHIBITORS USEFUL IN PROLONGING SURVIVAL OF MAM-MALIANS WITH CONGESTIVE HEART FAILURE

## BACKGROUND OF THE INVENTION

The use of the angiotensin converting enzyme (ACE) inhibitor captopril to prolong survival of congestive heart failure (CHF) rats has been evaluated by M.A. Pfeffer, et al. [Circulation, 72, No. 2, 406-4l2 (l985)]. This study reports that CHF rats having small infarcts (5%-l9.99%) and moderate infarcts (20%-39.9%) which were treated with captopril demonstrated reduced mortality, but that a long term beneficial effect was not sustained with captopril in CHF rats having large infarcts (40%). From this study, it appears that survival of rats with small infarcts after one year was about 70%; survival of rats with moderate infarcts after one year was about 50%; and, survival of rats with large infarcts after one year was about l8%.

## SUMMARY OF THE INVENTION

It has now been found that treating CHF rats having large infarcts with enalapril, enalaprilat, or lisinopril significantly prolongs their survival.

Enalapril, enalaprilat, lisinopril, and related carboxyalkyl dipeptide derivatives as well as methods for their preparation are disclosed in U.S. Patents 4,374,829 and 4,472,380 which are incorporated herein by reference. Enalapril is the chemical compound N-[l(S)-ethoxycarbonyl-3-phenylpropyl]-L-alanyl-L-proline; enalaprilat is the chemical compound N-[l(S)-carboxy-3-phenylpropyl]-L-alanyl-L-proline; and, lisinopril is the chemical compound Nα-[l(S)-carboxy-3-phenylpropyl]-L-lysyl-L-proline.

## DETAILED DESCRIPTION OF THE INVENTION

### ASSAY METHOD

### PROTOCOL

Commercially obtained male Sprague-Dawley rats ranging in age from l6 to 20 weeks, were housed in our animal facility for at least 2 weeks before operation. The intramyocardial artery was ligated (or a sham operation) was performed in each by a well-known method. Ten (l0) days after the operative procedure all survivors were briefly anesthetized with ether and electrocardiograms were obtained. At the time of anesthesia, each rat was identified and a balanced-block randomization process was used to allocate animals to either tap water or enalapril (in drinking water) therapy groups. This randomization process was stratified according to three arbitrary electrocardiographic groups. The first group had a normal QRS complex without evidence of a pathologic Q wave in limb leads I or aVL; the second group had a Q wave (<l mV) in either of leads I or a VL, yet maintained a precordial R wave total of l0 mV or greater; and the third group had both abnormal limb-lead Q waves and a sum of precordial R waves that was l0 mV or less. This electrocardiographic classification system was used only to maintain a balance of rats with and without infarction in the placebo and enalapril groups, not for the subsequent survival analysis, which was performed with the histologic determinations of the size of myocardial infarction only.

Animals (2-3) were housed in clear polyethylene cages (up to a maximum of four rats per cage) and placed according to therapy assignment and time of entry into the study. Twenty-five animals were allowed free access to food and the assigned therapy (water or enalapril,≈l.0 mg/kg/day in the drinking water). The room was temperature and humidity controlled and had a l2 hour light-dark cycle. The cages were maintained on two racks, each of which abutted one side of a double-sided laminar flow unit (Germ Free Laboratories). Cages with animals assigned to tap water or enalapril were placed on the rack. An additional 50 rats with the sham operation were selected from the same group used for this study and maintained concurrently with the study animals. Twenty-five received tap water and twenty-five received enalapril. Each animal was followed until death or for up to l year after the operative procedure.

Cages were inspected for dead animals daily on weekdays, weekends and holidays. The date of each death was noted and the carcass was weighed and a gross post-mortem examination was performed on many of the rats. The heart was removed and placed in formalin. All survivors at l year after infarction were anesthetized with ether and the heart of each was arrested with intravenous KCl. Blood samples were also taken from the survivors to determine drug and ACE inhibitor levels.

### PATHOLOGY

The formalin-fixed heart was examined for infarcted areas. The atria and great vessels were trimmed from the ventricles. In most experiments, the right ventricular free wall was dissected from the left ventricle and both ventricles were weighed separately. The left ventricle was dehydrated in alcohol, cleared in xylene and embedded in paraffin. Transverse 50 mm sections were cut from the apex to the base of the left ventricle. Several sections were mounted and stained with Masson's trichrome from which hematoxylin was omitted to provide a clearer discrimination between fibrous scar and muscle.

A slide of each section then was projected onto a screen for the planimetric measurement (Zeiss videoplan) of size of infarction. The endocardial and epicardial circumferences as well as the lengths of scar for each of these surfaces were measured for each section. The lengths of scar and of circumference were summed separately for each of the epicardial an endocardial surfaces and the sums were expressed as a ratio of scar length to ventricular circumference for each surface. These two ratios were then averaged and expressed as a percentage to define size of infarction. Since serial sections of the entire left ventricle were evaluated, the size of in farction determined represents the percent of the left ventricular endocardial and epicardial surface areas occupied by fibrous scar tissue.

## ANALYSIS

Comparisons of survival in untreated and enalapril treated rats were performed separately by log-rank tests for all of the rats with and without infarction. No analyses were performed in the two groups based on size of infarction.

## RESULTS

The results obtained for rats with large infarcts; i.e., ≥ 40%, are set forth in Figure I. As shown in Figure I, about 25% of rats having large infarcts survived for I year upon being treated with I mg/kg/day of enalapril. As noted earlier, the one year survival of rats with large infarcts after treatment with about 200 mg/kg/day of captopril was about I8%. Thus, use of enalapril at substantially lower doses then captopril results in prolonging CHF survival by about 50% more than captopril. More significantly, these results indicate enalapril is an effective therapeutic agent in prolonging survival of CHF mammalians, particularly those who have experienced large infarcts and that similar benefits would be expected by treating humans affected with CHF.

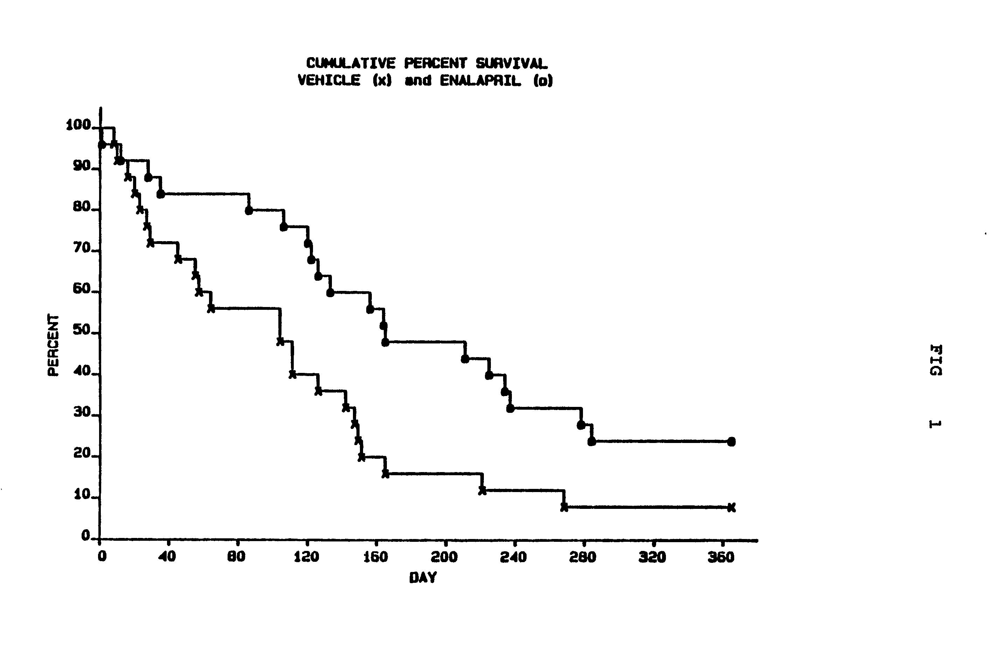

## Claims

1. The use of an angiotensin converting enzyme inhibitor selected from the group consisting of enalapril; enalaprilat and lisinopril, for the manufacture of a medicament useful for prolonging survival of mammalians with congestive heart failure.

2. The use as claimed in Claim 1 wherein the congestive heart failure is an infarct ≥ 40%.

3. The use as claimed in Claim I wherein the angiotensin converting enzyme is enalapril.

4. The use of an angiotensin converting enzyme selected from the group consisting of enalapril, enalaprilat and lisinopril, for the manufacture of a medicament useful for prolonging survival of mammalians with congestive heart failure having an infarct ≥ 40%.

5. The use as claimed in Claim 4 wherein the angiotensin converting enzyme is enalapril.

CUMULATIVE PERCENT SURVIVAL
VEHICLE (x) and ENALAPRIL (o)

0 241 201

FIG 1